(19) Europäisches Patentamt
European Patent Office
Office européen des brevets

(11) EP 0 564 672 B2

(12) NEUE EUROPÄISCHE PATENTSCHRIFT

(45) Veröffentlichungstag und Bekanntmachung des Hinweises auf die Entscheidung über den Einspruch:
06.05.1999 Patentblatt 1999/18

(45) Hinweis auf die Patenterteilung:
19.06.1996 Patentblatt 1996/25

(51) Int. Cl.⁶: $A61M\ 1/28$, $A61K\ 33/14$, $A61K\ 38/02$
// ($A61K38/02$, $33{:}00$, $31{:}70$, $31{:}415$, $31{:}405$, $31{:}40$, $31{:}195$, $31{:}19$)

(21) Anmeldenummer: 92105911.9

(22) Anmeldetag: 06.04.1992

(54) **Wässrige Peritonealdialyse-Lösung**

Aqueous solution for peritoneal dialysis

Solution aqueuse pour la dialyse péritonéale

(84) Benannte Vertragsstaaten:
AT BE CH DE DK ES FR GB GR IT LI LU MC NL PT SE

(43) Veröffentlichungstag der Anmeldung:
13.10.1993 Patentblatt 1993/41

(73) Patentinhaber:
BAXTER INTERNATIONAL INC.
Deerfield, IL 60015-4633 (US)

(72) Erfinder:
• Du Moulin, Axel, Dr.
W-8062 Markt-Indersdorf (DE)
• Müller-Derlich, Jutta, Dr.
W-8000 München 19 (DE)

(74) Vertreter:
Dost, Wolfgang, Dr.rer.nat., Dipl.-Chem. et al
Patent- und Rechtsanwälte
Bardehle . Pagenberg . Dost . Altenburg .
Geissler . Isenbruck
Postfach 86 06 20
81633 München (DE)

(56) Entgegenhaltungen:
EP-A- 0 022 922          EP-A- 0 086 553
EP-A- 0 161 471          EP-A- 0 347 714
EP-A- 0 399 549          EP-A- 0 437 274
WO-A-87/01286            WO-A-91/08008
US-A- 4 465 488          US-A- 4 959 175

• "Amino acids for peritoneal dialysis: technical and metabolic implications", Linholm et al., Peritoneal Dialysis - Proc. 2nd International course, 1986, Seiten 149 - 154
• "Sodium Lactate and Other Buffers for Peritoneal Dialysis", Biasoli et al., Contemporary Dialysis, Okt. 1982, Seiten 46 - 49
• "Bicarbonate Buffer for CAPD Solution", Feriani et al., Vol. XXXI, Trans. Am. Soc., Artif. Intern. Organs, 1985, Seiten 668 - 672

EP 0 564 672 B2

**Beschreibung**

Gegenstand der vorliegenden Erfindung ist eine wässrige Peritonealdialyse-Lösung, die unmittelbar vor Gebrauch aus zwei Einzel-Lösungen erhalten wird und eine osmotisch wirksame Substanz und Bicarbonationen enthält.

Bei Patienten mit akuter oder chronischer Niereninsuffizienz muß die eingeschränkte Nierenfunktion durch alternative Verfahren kompensiert werden. Derartige alternative Verfahren sind die Hämodialyse und die Peritonealdialyse. Bei der sog. CAPD (kontinuierlichen ambulanten Peritoneal-Dialyse) wird die Peritonealhöhle von nierenkranken Patienten mehrfach täglich mit einer frischben Peritonealdialyse-Lösung gefüllt. Bei dieser Art von Dialyse findet die Entgiftung und Entwässerung über die den gesamten Bauchraum auskleidende Peritonealmembran statt. Das Peritoneum bildet beim Stoffaustausch eine semipermeable Membran, durch die gelöste Stoffe im Sinne der Diffusion durchtreten. Einzelheiten dieses Stofftransportes sind noch nicht endgültig aufgeklärt. Innerhalb von zwei bis drei Stunden kommt es durch Diffusion zu einem Konzentrationsanstieg der harnpflichtigen Substanzen in der frisch eingefüllten Peritonealdialyse-Lösung. Gleichzeitig erfolgt entsprechend dem osmotischen Gleichgewicht der Flüssigkeitsentzug durch Ultrafiltration. Die Peritonealdialyse-Lösung verbleibt über 4 - 8 Stunden im Bauchraum und wird danach durch einen Katheter nach außen abgelassen. Die Prozedur erfolgt in der Regel viermal täglich und dauert etwa 30 - 40 Minuten. Beim Wechsel der Peritonealdialyse-Lösung ist eine Diskonnektion zwischen Katheter und einem zum Peritonealdialyse-Beutel verlängernden Schlauchsystem notwendig.

Die bisher insbesondere in der kontinuierlichen ambulanten Peritonealdialyse verwendeten Dialyse-Lösungen haben aus Stabilitätsgründen meist einen sauren pH-Wert im Bereich von 5,2 - 5,5. Solche sauren Dialyse-Lösungen können zu Schädigungen des Peritoneums, zu Reizungen des Abwehrsystems des Körpers und zu Schmerzen in der Bauchhöhle führen. So ist beispielsweise in der DE-A-38 21 043 eine solche saure Dialysier- und Spüllösung zur intraperitonealen Verabreichung beschrieben. Allerdings gibt es auch schon Peritonealdialyse-Lösungen, deren pH-Wert zwischen 7,0 und 7,6 liegt. Eine solche Lösung, die aus zwei Einzel-Lösungen besteht, ist aus der EP-A-0 399 549 bekannt. Die eine Einzel-Lösung enthält eine osmotisch wirksame Substanz und hat einen pH-Wert von 5,5 bis 6,2 und die andere Einzel-Lösung enthält Bicarbonationen und hat einen pH-Wert von 7,0 bis 7,6. Hier ergibt sich das Problern, daß die beten Einzel-Losungen keine ausreichende Stabilität, insbesondere ihres pH-Wertes aufweisen. Darüberhinaus laßt sich der pH-Wert bei den Einzel-Lösungen nur schwierig einstellen.

WO-A-91/08008 offenbart eine Peritonealdialyse-Lösung, die vor Gebrauch aus zwei Einzellösungen hergestellt wird, wobei die erste Einzel-Lösung eine osmotisch wirksame Substanz enthalt und die zweite Einzel-Lösung L-Histidin enthält, das die Peritonealdialyse-Lösung auf einen pH-Wert von mindestens 6,5 puffert.

Die dieser Erfindung zugrundeliegende Aufgabe bestand somit darin, eine Peritonealdialyse-Lösung mit physiologischer Zusammensetzung in Bezug auf pH-Wert, Bicarbonationen-Konzentration und $pCO_2$ bereitzustellen, bei der die oben genannten pH-Probleme nicht auftreten.

Gegenstand der vorliegenden Erfindung ist eine wässrige Peritonealdialyse-Lösung gemäß Patentanspruch 1.

Vorteilhafte Ausgestaltungen sind in den Ansprüchen 2 bis 7 beschrieben.

In der beanspruchten Peritonealdialyse-Lösung wird eine physiologische Zusammensetzung in Bezug auf pH-Wert, Bicarbonationen-Konzentration und $pCO_2$ erreicht und infolge der Pufferwirkung der Allionen der Mono- oder Dicarbonsäuren bzw. der Aminosäuren oder Peptide läßt sich der pH-Wert in den Einzel-Lösungen sehr leicht genau einstellen.

Der pH-Wert der ersten Einzel-Lösung beträgt von 4,5 bis 5,8, bevorzugt 4,8 bis 5,6, insbesondere 5,0 bis 5,5.

Als Anionen der Mono- und/oder Dicarbonsäuren in der ersten Einzel-Lösung werden beispielsweise Lactat, Acetat, Citrat oder Formiat verwendet, vorzugsweise Lactat oder Acetat, insbesondere Lactat.

Als osmotisch wirksame Substanz in der ersten Einzel-Lösung kommen beispielsweise Glukose, Galactose, Polyglukose oder Fructose sowie Polyole wie Glycerin oder Sorbit in Frage. Vorzugsweise wird Glukose oder Galactose verwendet, insbesondere Glukose.

Der pH-Wert der zweiten Einzel-Lösung beträgtvon 7,2 bis 10,0, vorzugsweise 7,3 bis 8,0, insbesondere 7,4 bis 7,6.

Die zweite Einzel-Lösung enthält Mischungen von Aminosäuren oder einzelne Aminosäuren bzw. Mischungen von Peptiden bzw. einzelne Peptide. Die Auswahl der Aminosäure oder der Aminosäuren-Mischung bzw. des Peptids oder der Peptid-Mischung unterliegt keiner Beschränkung. Jede der zwanzig bekannten Aminosäuren ist gleichermaßen als Einzel-Komponente oder innerhalb einer Mischung bevorzugt. Als Peptide werden z.B. Hydrolysate aus Milchproteinen verwendet.

Eingestellt werden die pH-Werte der ersten und zweiten Einzel-Lösung mit physiologisch verträglichen Säuren, wie z.B. HCl, Milchsäure oder Essigsäure, vorzugsweise mit HCl.

Die beiden Einzel-Lösungen werden im allgemeinen im Verhältnis von 3:1 bis 1:3, vorzugsweise 1:1 bis 1:2, miteinander vermischt.

Die folgenden Angaben beziehen sich auf die Zusammensetzung der Peritonealdialyse-Lösung, d.h. nach Vereinigung der beiden Einzel-Lösungen.

Die Konzentration der osmotisch wirksamen Substanz in der Peritonealdialyse-Lösung beträgt 0,5 % bis 10 %, vorzugsweise 0,8 % bis 7 %, besonders bevorzugt 1 % bis 5 %.

Die Konzentration der Anionen der Mono- und/oder Dicarbonsäuren in der Peritonealdialyse-Lösung beträgt 5 bis 100 mmol/l, vorzugsweise 10 bis 60 mmol/l, insbesondere 15 bis 40 mmol/l.

Die Konzentration der Aminosäure-Komponente bzw. der Peptid-Komponente in der Peritonealdialyse-Lösung beträgtvon 0,05 Gew.-% bis 2 Gew.-%, vorzugsweise 0,1 Gew.-% bis 1 Gew.-%, insbesondere 0,2 Gew.-% bis 0,5 Gew.-%.

Die Konzentration der osmotisch wirksamen Substanz wird entsprechend dem Beitrag o.g. Komponenten zur osmotischen Wirksamkeit verringert.

Die Peritonealdialyse-Lösung enthält bevorzugt noch Ionen ausgewählt aus $Na^+$, $Cl^-$, $Ca^{2+}$, $Mg^{2+}$ oder $K^+$. Die Konzentrationen dieser Ionen sind aus dem Stand der Technik, wie der EP-A-0 399 549 oder der EP-A-0 347 714, bekannt.

Die Peritonealdialyse-Lösung kann ferner vorzugsweise übliche Zusätze, beispielsweise Vitamine, den Proteinstoffwechsel beeinflußende Hormone, Fettsäuren und/oder Fette enthalten.

Die Peritonealdialyse-Lösung hat erfindungsgemäß folgende Parameter: 23 - 26 mmol/l Bicarbonationen, 25 bis 70 mm $pCO_2$, pH-Wert 7,2 bis 7,6.

Die vorgenannten Parameter der Peritonealdialyse-Lösung sind über die Zusammensetzungen der beiden Einzel-Losungen steuerbar, so daß die beiden Einzel-Lösungen exakt aufeinander abgestimrnt sein müssen.

Der jeweils erforderliche pH-Wert der zweiten Einzel-Losung, $pH_{II}$, wird durch folgende Gleichung definiert, wobei deutlich wird, daß der $pH_{II}$ von den Konzentrationen und den jeweiligen pK-Werten der Substanzen als auch vom üblicherweise vorgegebenen $pH_I$ abhängt.

$$pH_{II}=pK_{II}+\log\left[\frac{C_{IIM}(10^{pH_M-pK_{II}}+1)}{C_{IIM}-D(10^{pH_M-pK_{II}}+1)}-1\right] \tag{1}$$

$$\text{wobei } D=C_{IM}\left[\frac{1}{10^{pH_I-pK_I}+1}-\frac{1}{10^{pH_M-pK_I}+1}\right] \tag{2}$$

Bei Vorliegen nur jeweils einer dissoziierenden Substanz in den beiden Einzel-Losungen, kann der pK-Wert der Carboxylgruppe für die erste Einzel-Lösung bzw. der Aminogruppe für die zweite Einzel-Lösung eingesetzt werden. Bei Vorliegen von zwei oder mehr dissoziierenden Gruppen in den beiden Einzel-Lösungen ist der Misch-pK über alle dissoziierenden Gruppen für jede der beiden Einzel-Lösungen nach folgender Gleichung zu berechnen:

$$pK=\frac{\sum_{i=1}^{i=n} C_i pK_i}{\sum_{i=1}^{i=n} C_i} \tag{3}$$

Bei z.B. zwei dissoziierenden Gruppen pro Substanz wird der $\overline{pK}$ wie folgt berechnet:

$$\overline{pK}=\frac{pK_1+pK_2}{2} \tag{4}$$

Wobei im Bereich pH $\leqq$ 6,5 nur die anionischen Gruppen und im Bereich pH > 6,5 nur die kationischen Gruppen berücksichtigtwerden. Bei Histidin wird daher bei der Berechnung des $\overline{pK}$ der pK-Wert der Carboxylgruppe nicht berücksichtigt.

Die Abkürzungen haben folgende Bedeutungen:

$pH_I$ = pH-Wert der ersten Einzel-Lösung
$pH_{II}$ = pH-Wert der zweiten Einzel-Lösung
$c_{IIM}$ = Summe der molaren Konzentrationen in der Peritonealdialyse-Lösung von Bicarbonationen plus Amino-säure-Komponente oder Peptid-Komponente aus der zweiten Einzel-Lösung

$pK_{II}$ = pK-Wert der Komponenten der zweiten Einzel-Lösung (berechnet nach Formel (3), falls erforderlich)

$pH_M$ = gewünschter pH-Wert der Peritonealdialyse-Lösung nach Vereinigung der beiden Einzel-Lösungen

$c_{IM}$ = Summe der molaren Konzentrationen in der Peritonealdialyse-Lösung der Anionen der Mono- und/oder Dicarbonsäuren aus der ersten Einzel-Lösung

$pK_I$ = pK-Wert der Carbonsäuren der ersten Einzel-Lösung (berechnet nach Formel (3), falls erforderlich)

$c_i$ = Konzentration der Substanz i

$pK_i$ = pK-Wert der Substanz i

$pK_1$ = pK-Wert der dissoziierenden Gruppe 1

$pK_2$ = pK-Wert der dissoziierenden Gruppe 2

$\overline{pK}$ = mittlerer pK-Wert für Substanzen mit zwei dissoziierenden Gruppen Der einzustellende pH-Wert der zweiten Einzel-Lösung wird mit der angegebenen Gleichung (1) ausgerechnet. Dazu ist die Berechnung der pK-Werte beider Einzel-Lösungen notwendig, was mit Gleichung (3) erfolgt. Weiter müssen bekannt sein bzw. müssen vorher festgelegt werden: Der pH-Wert der Peritonealdialyse-Lösung (nach Vereinigung der beiden Einzel-Lösungen); der pH-Wert der, die Anionen der Mono- und/oder Dicarbonsäuren enthaltenden ersten Einzel-Lösung sowie die Konzentrationen in der Peritonealdialyse-Lösung deraus der ersten bzw. zweiten Einzel-Lösung stammenden Carbonsäuren bzw. des Bicarbonats und der Amionosäuren oder der Peptide. Mit diesen Größen wird D in Gleichung (2) ausgerechnet und in Gleichung (1) eingesetzt. Mit Hilfe des obigen Gleichungssystems ist es möglich, für jede Aminosäure oder jede Aminosäuren-Mischung bzw. jedes Peptid oder jede Peptid-Mischung den in der zweiten Einzel-Lösung einzustellenden pH-Wert zu bestimmen, um nach Mischung der beiden Einzel-Lösungen in der Peritonealdialyse-Lösung einen gewünschten pH-Wert zu erreichen.

Was oben für die Ausrechnung des pH-Wertes der zweiten Einzel-Lösung gesagt ist, gilt sinngemäß auch für die erste Einzel-Lösung bei bekannten Parametern der zweiten Einzel-Lösung und der Peritonealdialyse-Lösung.

Da die zweite Einzel-Lösung Bicarbonat enthält, erfolgt die Einstellung des pH-Wertes in der zweiten Einzel-Lösung, um möglichst wenig $CO_2$ entweichen zu lassen, unter Luftabschluß.

Die Handhabung der erfindungsgemäßen Peritonealdialyse-Lösung kann nach bekannten Verfahren erfolgen, z.B. gemäß der EP-A-0 161 471. Die Sterilisierung und Lagerung der beiden Einzel-Lösungen erfolgt vorzugsweise in einem Doppelkammer-Beutel. Vor Gebrauch der Peritonealdialyse-Lösung kann eine sterile Vermischung der beiden Einzel-Lösungen auf sehr einfache Art durch Öffnen eines Ventils zwischen den Kammern erreicht werden. Da diese Beutel meist aus Kunststoff sind, ist aufderen Gasundurchlässigkeit, insbesonderefür $CO_2$, zu achten. Daher sind diese Beutel nach außen hin mit Aluminiumfolie abgedichtet.

Eine Alternative zum Doppelkammer-Beutel besteht darin, die beiden Einzel-Lösungen in getrennten Behältnissen (Beutel, Flaschen) zu sterilisieren und aufzubewahren. Das vor Gebrauch notwendige Vermischen der beiden Einzel-Lösungen erfolgt vorzugsweise über ein geeignetes Verbindungssystem (Schlauchsystem).

Aus praktischen Erwägungen enthält die bicarbonathaltige zweite Einzel-Lösung kein $Ca^{2+}$, um die Ausfällung von $CaCO_3$ zu vermeiden.

Die erfindungsgemäße Peritonealdialyse-Lösungen findet üblicherweise in der Peritonealdialyse Verwendung. Sie wird jedoch auch in der Hämodialyse eingesetzt.

Im folgenden wird die Erfindung anhand der Abbildungen beschrieben: Abbildung (A) zeigt die Abhängigkeit des einzustellenden pH-Wertes in der zweiten Einzel-Lösung von der Konzentration in der Peritonealdialyse-Lösung ($c_{IIM}$) und dem pK-Wert der Substanzen aus der zweiten Einzel-Lösung, um nach Vermischen mit der ersten Einzel-Lösung (pH = 5,2) eine Peritonealdialyse-Lösung mit einem pH-Wert von 7,4 zu erreichen. Der pK-Wert der ersten Einzel-Lösung ist $pK_I$ = 4,0 und die Konzentration der Mono- und/oder Dicarbonsäure-Anionen in der ersten Einzel-Lösung $c_I$ = 70 mmol/l.

Wie Abbildung (A) zeigt, ist der in der zweiten Einzel-Lösung einzustellende pH-Wert abhängig von der Konzentration der Substanzen aus der zweiten Einzel-Lösung. Bei höheren Konzentrationen und abhängig vom pK-Wert ist der pH-Wert relativ unempfindlich gegen kleine Konzentrationsänderungen. Bei geringen Konzentrationen wirken sich jedoch kleine Konzentrationsänderungen sehr stark auf den erforderlichen $pH_{II}$ aus. Herstellungstechnisch werden daher Konzentrationen im oberen Bereich bevorzugt.

Abbildung (B) zeigt die Abhängigkeit des einzustellenden pH-Wertes in der zweiten Einzel-Lösung ($pH_{II}$) vom pK-Wert der Substanzen aus der zweiten Einzel-Lösung und deren Konzentration in der Peritonealdialyse-Lösung, um nach Mischung mit der ersten Einzel-Lösung (pH = 5,2) eine Peritonealdialyse-Lösung mit einem pH-Wert von 7,4 zu erreichen. Der pK-Wert der ersten Einzel-Lösung ist $pK_I$ = 4,0 und die Konzentration der Mono- und/oder Dicarbonsäure-Anionen in der ersten Einzel-Lösung ist $c_I$ = 70 mmol/l.

Aus Abbildung (B) geht deutlich hervor, daß mit Ausnahme des Minimums der Kurven, zwei pK-Werte zum selben $pH_{II}$ führen. Ebenfalls wird deutlich, daß bei einem bestimmten $pK_{II}$-Wert die gewählte Konzentration entscheidend ist, ob ein pH-Wert einstellbar ist, so daß sich der gewünschte $pH_M$-Wert nach Mischung beider Einzel-Lösungen einstellen läßt.

Das folgende Beispiel erläutert die Erfindung.

Die Peritonealdialyse-Lösung besteht zunächst aus zwei Einzel-Lösungen, die in getrennten Kompartimenten eines Doppelkammer-Beutels vorliegen. Dabei handelt es sich zum einen um eine erste Einzel-Lösung enthaltend Glukose, Lactat und Elektrolyte und zum anderen um eine zweite Einzel-Lösung enthaltend Bicarbonationen und 15 verschiedene Aminosäuren. Unmittelbar vor Gebrauch werden beide Einzel-Lösungen zu einer Peritonealdialyse-Lösung vermischt. Für diese Mischung ist ein physiologischer pH-Wert von 7,20 bis 7,60 bei 37 °C festgelegt worden, wobei im Mittel ein physiologischer pH-Wert von 7,4 erreicht wird. Dabei soll die glukosehaltige erste Einzel-Lösung pH = 5,00, 5,20 bzw. 5,50 (vor Autoklavierung) haben und gemäß diesen Vorgaben soll der pH-Wert für die zweite Einzel-Lösung rechnerisch ermittelt werden und dann mittels Säure eingestellt werden.

Beide Einzel-Lösungen sind zudem so konzipiert, daß die gebrauchsfertige Peritonealdialyse-Lösung 24 mmol/l Bicarbonat aufweist.

Erste Einzel-Lösung (Glukose/Lactat-Lösung):

2,96 %, 5,38% oder 9,63 % Glukose
40 mmol/l Na-Lactat ($pK_s = 3,86$)
4,7 mmol/l $CaCl_2$
2 mmol/l $MgCl_2$
258 mmol/l NaCl

Zweite Einzel-Lösung (Aminosäure/Bicarbonat-Lösung):

40,44 mmol/l Na-Bicarbonat ($pK_s = 5,98$)
0,4 Gew-% (31,25 mmol/l) Aminosäuren

Peritonealdialyse-Lösung (Mischung der beiden Einzel-Lösungen):

0,75 l erste Einzel-Lösung + 1,25 l zweite Einzel-Lösung
1,11 %, 2,02 % oder 3,61 % Glukose
0,25 Gew-% Aminosäuren
15 mmol/l Na-Lactat
24 mmol/l Na-Bicarbonat
(1,28 mmol/l $CO_2$)
1,75 mmol/l $CaCl_2$
0,75 mmol/l $MgCl_2$
97 mmol/l NaCl

Verwendete Aminosäuren:

Valin 2,70 mmol/l ($pK_s = 9,62$)
Leucin 1,77 mmol/l ($pK_s = 9,6$)
Isoleucin 1,47 mmol/l ($pK_s = 9,62$)
Methionin 1,30 mmol/l ($pK_s = 9,21$)
Lysin/HCl 0,95 mmol/l ($\overline{pK_s} = 9,74$)
Histidin 1,04 mmol/l ($\overline{pK_s} = 7,5$)
Threonin 1,24 mmol/l ($pK_s = 9,12$)
Phenylalanin 0,78 mmol/l ($pK_s = 9,13$)
Tryptophan 0,30 mmol/l ($pK_s = 9,39$)

Arginin 1,40 mmol/l ($\overline{pK_S}$ = 10,76)
Alanin 2,42 mmol/l ($pK_s$ = 9,69)
Prolin 1,16 mmol/l ($pK_s$ = 10,6)
Glycin 1,54 mmol/l ($pK_s$ = 9,6)
Serin 1,10 mmol/l ($pKs$ = 9,15)
Tyrosin 0,36 mmol/l ($pK_s$ = 9,11)

Zuerst werden die pK-Werte gemäß Gleichung (3) getrennt für die erste und zweite Einzel-Lösung ausgerechnet. Mit Gleichung (2) und den obigen Angaben für die erste Einzel-Lösung wird D ausgerechnet. Da nun alle Größen für das Lösen von Gleichung (1) bekannt sind, kann der pH-Wert für die zweite Einzel-Lösung mit dieser Gleichung ausgerechnet werden. Eingestellt wird der pH-Wert mit 1 mol/l HCl.

Die pH-Werte der ersten und der zweiten Einzel-Lösung wurden jeweils vor dem Antoklavieren bestimmt. Nach dem Autoklavieren wurden beide zu einer gebrauchsfertigen CAPD-Lösung gemischt. Die Anzahl der untersuchten Beutel je Versuchsserie war 8 oder 9. Es wurden Doppelwert-Bestimmungen bei allen Beuteln vorgenommen. Bei der gebrauchsfertigen Peritonealdialyse-Lösung wurde ferner der $pCO_2$-Wert bestimmt. Das Ergebnis ist der folgenden Tabelle zu entnehmen:

| Erste Einzel-Lösung pH (vorgegben) | Zweite Einzel-Lösung pH (eingestellt) | CAPD-Lösung nach Vermischen der beiden Einzel-Lösungen (37 °C) | | $pCO_2$ (mmHg) |
|---|---|---|---|---|
| | | pH (Soll) | pH (Ist) | |
| 5.00 | 7.25 | 7.20 | 7.25 | 62,7 |
| 5.20 | 7.32 | 7.40 | 7.41 | 38,8 |
| 5.50 | 7.57 | 7,60 | 7.59 | 28,1 |

Wie aus obiger Tabelle zu sehen ist, werden in der Praxis die angestrebten pH-Werte der Peritonealdialyse-Lösung in sehr guter Näherung erhalten. Die Abweichung beträgt beim ersten Wert 0,05 pH-Einheiten und bei den beiden anderen Werten nur jeweils 0,01 pH-Einheiten. Voraussetzung für die gute Übereinstimmung zwischen angetrebtem und erhaltenem pH-Wert in der Peritonealdialyse-Lösung ist allerdings, daß in den Einzel-Lösungen die pH-Werte exakt eingestellt werden können und stabil bleiben.

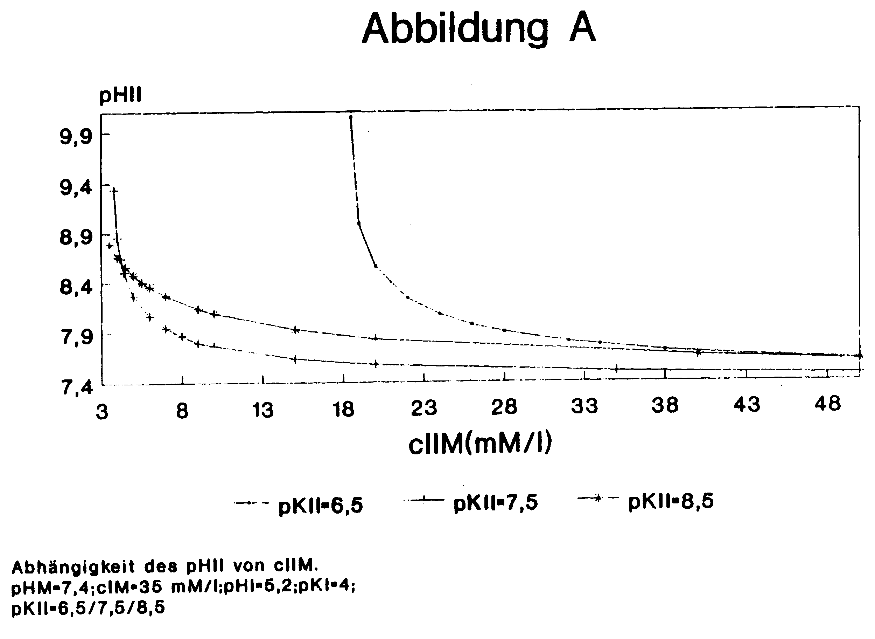

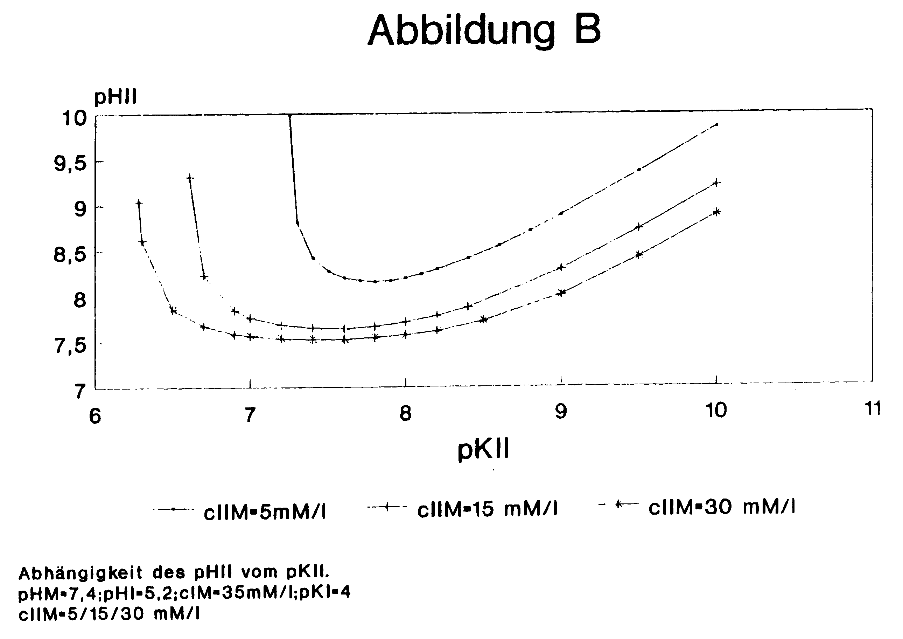

**Patentansprüche**

1. Wäßrige Peritonealdialyse-Lösung, wobei diese Losung unmittelbar vor Gebrauch aus zwei Einzel-Losungen erhalten wird,
   **dadurch gekennzeichnet**, daß

   - die erste Einzel-Lösung eine osmotisch wirksame Substanz und Anionen von Mono- und/oder Dicarbonsäuren enthält und einen pH-Wert von 4,5 bis 5,8 besitzt,

   - die zweite Einzel-Lösung Bicarbonationen und eine Aminosäure-Komponente oder eine Peptid-Komponente enthält und einen pH-Wert von 7,2 bis 10,0 besitzt, und

   - die gebrauchsfertige Losung 23 - 26 mmol/l Bicarbonationen enthält sowie einen $CO_2$-Partialdruck von 25 - 70 mm Hg und einen pH-Wert von 7,2 bis 7,6 besitzt.

2. Lösung nach Anspruch 1, dadurch gekennzeichnet, daß die beiden Einzel-Lösungen in getrennten Kompartimenten eines Doppelkammer-Beutels vorliegen.

3. Lösung nach Anspruch 1 oder 2, dadurch gekennzeichnet, daß die Aminosäure-Komponente in der zweiten Einzel-Lösung eine einzelne Aminosäure oder ein Gemisch aus mindestens zwei Aminosäuren ist.

4. Lösung nach Anspruch 1 oder 2, dadurch gekennzeichnet, daß die Peptid-Komponente in der zweiten Einzel-Lösung ein einzelnes Peptid oder ein Gemisch aus mindestens zwei Peptiden ist.

5. Lösung nach einem der Ansprüche 1 - 4, dadurch gekennzeichnet, daß die osmotisch wirksame Substanz in der ersten Einzel-Lösung Glukose ist.

6. Lösung nach einem der Ansprüche 1 - 5, dadurch gekennzeichnet, daß das in der ersten Einzel-Lösung enthaltene Carbonsäure-Anion Laktat ist.

## Claims

1. An aqueous peritoneal dialysis solution which is obtained immediately prior to administration from two individual solutions, **characterized in that** the first individual solution contains an osmotically active substance and anions of mono- and/or dicarboxylic acids and has a pH value of 4.5 to 5.8, that the second individual solution contains bicarbonate ions and an amino acid component or a peptide component and has a pH value of 7.2 to 10.0, and that the ready-to-use solution contains 23 to 26 mmoles/L bicarbonate ions and has a $CO_2$ partial pressure of 25 to 70 mmHg and a pH value of 7.2 to 7.6.

2. The solution according to claim 1, characterized in that the two individual solutions are available in separate compartments of a bag with two chambers.

3. The solution according to claim 1 or 2, characterized in that the amino acid component in the second individual solution is a single amino acid or a mixture of a minimum of two amino acids.

4. The solution according to claim 1 or 2, characterized in that the peptide component in the second individual solution is a single peptide or a mixture of a minimum of two peptides.

5. The solution according to any of claims 1 to 4, characterized in that the osmotically active substance in the first individual solution is glucose.

6. The solution according to any of claims 1 to 5, characterized in that the carboxylic acid anion in the first individual solution is lactate.

## Revendications

1. Solution aqueuse pour dialyse péritonéale, cette solution étant préparée immédiatement avant emploi à partir de deux solutions individuelles, caractérisée en ce que :

   - la première solution individuelle contient une substance à effet osmotique et des anions d'acides mono-et/ou dicarboxyliques, et a un pH de 4,5 à 5,8,
   - la deuxième solution individuelle contient des ions bicarbonate et un constituant acide aminé ou un constituant peptide, et a un pH de 7,2 à 10,0, et
   - la solution prête à l'emploi contient 23 à 26 mmol/l d'ions bicarbonate, et a une pression partielle de $CO_2$ de 3,33 à 9,33 kPa (25 à 70 mm Hg) et un pH de 7,2 à 7,6.

2. Solution seion la revendication 1, caractérisée en ce que les deux solutions individuelles sont présentes dans des compartiments distincts d'une poche à double compartiment.

3. Solution selon la revendication 1 ou 2, caractérisée en ce que le constituant acide aminé de la deuxième solution individuelle est un acide aminé individuel ou un mélange d'au moins deux acides aminés.

4. Solution selon la revendication 1 ou 2, caractérisée en ce que le constituant peptide de la deuxième solution individuelle est un peptide individuel ou un mélange d'au moins deux peptides.

5. Solution selon l'une des revendications 1 à 4, caractérisée en ce que la substance à effet osmotique de la première solution individuelle est le glucose.

6. Solution selon l'une des revendications 1 à 5, caractériséeen ce que l'anion acide carboxylique contenu dans la première solution individuelle est le lactate.

# Abbildung A

Abhängigkeit des pHII von cIIM.
pHM=7,4;cIM=35 mM/l;pHI=5,2;pKI=4;
pKII=6,5/7,5/8,5

EP 0 564 672 B2

# Abbildung B

Abhängigkeit des pHII vom pKII.
pHM=7,4;pHI=5,2;cIM=35mM/I;pKI=4
cIIM=5/15/30 mM/I